(19) Europäisches Patentamt
European Patent Office
Office européen des brevets

(11) Publication number: **0 276 740**
**A1**

(12) **EUROPEAN PATENT APPLICATION**

(21) Application number: **88100763.7**

(22) Date of filing: **20.01.88**

(51) Int. Cl.4: **C07K 9/00** , C07K 7/06 , A61K 37/02

(30) Priority: **28.01.87 GB 8701872**

(43) Date of publication of application: **03.08.88 Bulletin 88/31**

(84) Designated Contracting States: **AT BE CH DE ES FR GB GR IT LI LU NL SE**

(71) Applicant: **GRUPPO LEPETIT S.P.A.**
**23, Via G. Murat**
**I-20159 Milano(IT)**

(72) Inventor: **Malabarba, Adriano**
**5/A, Via Roma**
**I-20082 Binasco (MI)(IT)**
Inventor: **Trani, Aldo**
**11, Via Longhi**
**Milano(IT)**
Inventor: **Tarzia, Giorgio**
**3, Vicolo San Clemente**
**I-37100 Verona(IT)**

(74) Representative: **Macchetta, Francesco et al**
**Gruppo Lepetit S.p.A. Patent and Trademark Department 34, Via Roberto Lepetit**
**I-21040 Gerenzano (Varese)(IT)**

(54) **N15-alkyl and N15,N15-dialkyl derivates of teicoplanin compounds.**

(57) The present invention is directed to new $N^{15}$ mono-or di-alkyl derivatives of teicoplanin, a known antibiotic substance, or a pseudoaglycon or aglycon thereof. These compounds, as well as their pharmaceutically acceptable salts, posses antimicrobial activity in particular against gram positive bateria.

**EP 0 276 740 A1**

# $N^{15}$-ALKYL AND $N^{15}$,$N^{15}$-DIALKYL DERIVATIVES OF TEICOPLANIN COMPOUNDS.

$N^{15}$-alkyl and $N^{15}$,$N^{15}$-dialkyl derivatives of teicoplanin compounds having the following formula:

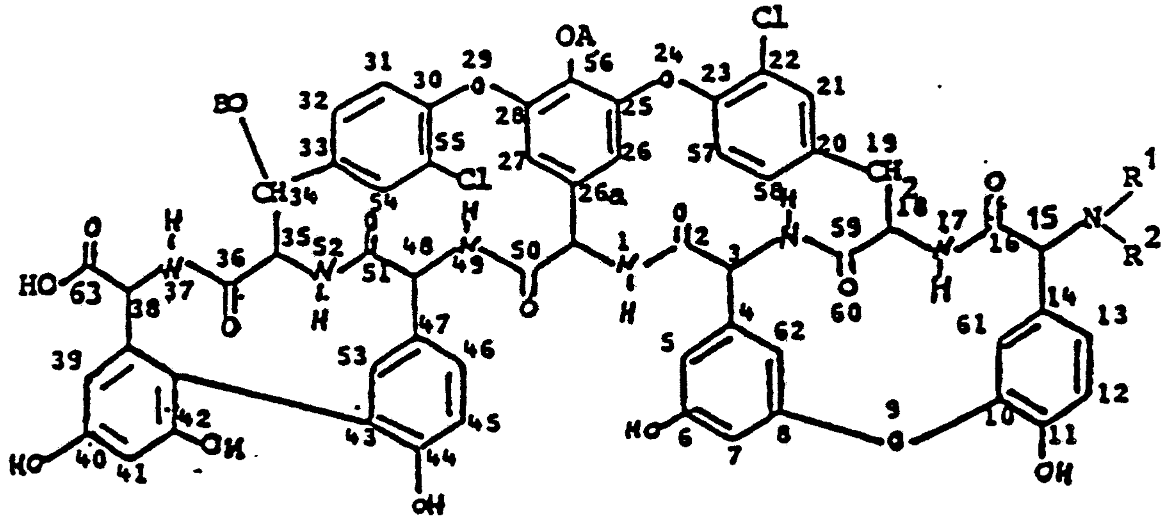

wherein

$R^1$ and $R^2$ independently represent hydrogen; $(C_1-C_{12})$alkyl; $(C_4-C_7)$cycloalkyl; cyano $(C_1-C_3)$alkyl; $-(CH_2)_n-OOC-(C_1-C_6)$alkyl; wherein n is an integer selected from 1, 2, 3 and 4; phenyl $(C_1-C_4)$alkyl wherein the phenyl group is optionally substituted in the position ortho, meta and/or para with 1 to 3 groups selected from $(C_1-C_4)$alkyl, nitro, bromo, chloro, iodo, $(C_1-C_4)$alkoxy, and phenyl; with the proviso that $R^1$ and $R^2$ may not simultaneously represent hydrogen and when one between $R^1$ and $R^2$ represents $(CH_2)_n-OOC-(C_1-C_6)$alkyl the other must represent hydrogen;

A represents hydrogen or -N[$(C_{10}-C_{11})$aliphatic acyl]-$\beta$-D-2-deoxy-2-amino-glucopyranosyl,

B represents hydrogen or N-acetyl-$\beta$-D-2-deoxy-2-amino-glucopyranosyl,

M represents hydrogen or $\alpha$-D-mannopyranosyl, with the proviso that B represents hydrogen only when A and M are simultaneously hydrogen and M represents hydrogen only when A is hydrogen; and the addition salts thereof.

Teicoplanin is the international non-proprietary name (INN) of the antibiotic substance formerly named teichomycin which is obtained by cultivating the strain Antinoplanes teichomyceticus nov. sp. ATCC 31121 in a culture medium containing assimilable sources of carbon, nitrogen and inorganic salts (see U.S. Patent No. 4,239,751).

According to the procedure described in the above cited patent an antibiotic complex containing Teichomycin $A_1$, $A_2$ and $A_3$ is recovered from the separated fermentation broth by extraction with a suitable water insoluble organic solvent and precipitation from the extracting solvent according to common procedures.

Teichomycin $A_2$, which is the major factor of the isolated antibiotic complex, is then separated from the other factors by means of column choromatography on Sephadex®.

British Patent Application Publication No. 2121401 discloses that antibiotic Teichomycin $A_2$ actually is a mixture of five closely related co-produced main components.

According to recent structural studies it is possible to represent teicoplanin $A_2$ (formerly Teichomycin $A_2$) main components 1, 2, 3, 4 and 5 by the above formula I wherein $R^1$ and $R^2$ are hydrogen, Y is hydroxy, A represents -N[$(C_{10}-C_{11})$aliphatic acyl] -$\beta$-D-2-deoxy-2-amino-glucopyranosyl, B represent N-acetyl-$\beta$-D-2-deoxy-2-amino-glucopyranolyl glucopyranosyl, M represents $\alpha$-D-manno-pyranosyl.

More particularly, in teicoplanin $A_2$ component 1, the [$(C_{10}-C_{11})$-aliphatic acyl] substituent represents Z-decenoyl, in teicoplanin $A_2$ component 2 represents 8-methyl-nonanoyl, in teicoplanin $A_2$ component 3 represents decanoyl, in teicoplanin $A_2$ component 4 represents 8-methyldecanoyl, in teicoplanin $A_2$ component 5 represents 9-methyldecanoyl.

All the sugar moieties, when present, are linked to the teicoplanin nucleus through O-glycosidic bonds.

In addition, it has been found that it is possible to transform teicoplanin, a pure factor thereof or a mixture of any of said factors in any proportion, into unitary antibiotic products by means of selective hydrolysis of one or two sugar moieties.

They are named antibiotic L 17054 and antibiotic L 17046 and are described in European Patent Application Publication No. 119575 and European Patent Application Publication No. 119574, respectively.

Preferred hydrolysis conditions for the production of antibiotic L 17054 are: 0.5 N hydrochloric acid at a temperature between 70°C and 90°C and for a time which is generally between 15 and 90 min.

Antibiotic L 17954 is represented by the above formula I wherein $R^1$, $R^2$ and A represent hydrogen, B represents N-acetyl-β-D-2-deoxy-2-amino-glucopyranosyl, M represents-α-D-mannopyranosyl wherein the sugar moieties are linked to the peptidic nucleus through an O-glycosidic bond.

Preferred hydrolysis conditions for the preparation of antibiotic L 17046 are: 1-3 N hydrochloric acid, at a temperature between 50° and 90°C and for a time which is generally between 30 and 60 min.

Antibiotic L 17046 is represented by the above formula I wherein $R^1$, $R^2$, A and M represent hydrogen atoms, and B is N-acetyl-β-D-2-deoxy-2-aminoglucopyranosyl wherein the sugar moiety is linked to the peptidic nucleus through an O-glycosidic bond.

The complete selective cleavage of all the sugar moieties of the teicoplanin compounds gives an aglycone molecule which is called antibiotic L 17392, or deglucoteicoplanin, and is represented by the above formula I wherein $R^1$, $R^2$, A, B, and M each individually represents a hydrogen group. This selective hydrolysis process is described in European patent application No. 84114558.4.

A substance having the same structural formula is disclosed in European Patent Application Publication No. 0090578 and is named antibiotic A 41030 factor B. This substance is obtained by means of a microbiological process which involves the fermentation of the strain Streptomyces virginiae NRRL 12525 or Streptomyces virginiae NRRL 15156 in a suitable medium, the isolation, purification and separation into its components of antibiotic A 41030, an antibiotic complex of at least seven factors, antibiotic A 41030 factor B, included.

All the above named compounds, i.e. teicoplanin, teicoplanin $A_2$ complex, teicoplanin $A_2$ component 1, teicoplanin $A_2$ component 2, teicoplanin $A_2$ component 3, teicoplanin $A_2$ component 4, teicoplanin $A_2$ component 5, antibiotic L 17054, antibiotic L 17046, antibiotic L 17392 and any mixture thereof in any proportion, are suitable starting materials for the preparation of the amide derivatives of the invention.

In the present specification "teicoplanin compound " or "teicoplanin starting material" is used to indicate any one of the above starting materials, i.e. teicoplanin as obtained according to U.S. patent 4,239,751, any further purification thereof, teicoplanin $A_2$ complex, a compound of the above formula I wherein $R^1$ and $R^2$ are hydrogen, A represents hydrogen or -N[($C_{10}$-$C_{11}$)aliphatic acyl]-β-D-2-deoxy-2-aminoglycopyranosyl, B represent hydrogen or N-acetyl-β-D-2-deoxy-2-amino-glucopyranosyl, M represents hydrogen or α-D-mannopyranosyl, with the proviso that B may represent hydrogen only when A and M are simultaneously hydrogen and M may represent hydrogen only when A is hydrogen, a salt thereof, or a mixture thereof in any proportion.

As used herein the term "alkyl", either alone or in combination with other substituents, includes both straight and branched hydrocarbon groups; more particularly, "($C_1$-$C_6$)alkyl" represents a straight or branched aliphatic hydrocarbon chain of 1 to 6 carbon atoms such as methyl, ethyl, propyl, 1-methylethyl, butyl, 1-methylpropyl, 1,1-dimethylethyl, pentyl, 1-methylbutyl, 2-methylbutyl, 1-hexanyl, 2-hexanyl, 3-hexanyl, 3,3-dimethyl-1-butanyl, 4-methyl-1-pentanyl and 3-methyl-1-pentanyl; likewise, "($C_1$-$C_4$) alkyl" represents a straight or branched hydrocarbon chain of 1 to 4 carbon atoms like those exemplified above.

The term "halogeno" represents an halogen atom selected from fluorine, chlorine, bromine and iodine.

The compound of the invention contains acidic and basic functions and can form, in addition to "internal salts", acid and base addition salts according to conventional procedures.

For the scope of the present description and claims the "internal salts" are encompassed by the definition of "non-salt" or "non-addition salt" form.

Preferred addition salts of the compounds of this invention are the pharmaceutically acceptable acid and/or base addition salts.

With the term "pharmaceutically acceptable acid and/or base addition salts" are intended those salts with acids and/or bases which from biological, manufacturing and formulation standpoint are compatible with the pharmaceutical practice as well as with the use in the animal growth promotion.

Representative and suitable acid addition salts of the compounds of formula I include those salts formed by standard reaction wtih both organic and inorganic acids such as, for example, hydrochloric, hydrobromic, sulfuric, phosphoric, acetic, trifluoroacetic, trichloracetic, succinic, citric, ascorbic, lactic, maleic, fumaric, palmitic, cholic, pamoic, mucic, glutamic, camphoric, glutaric, glycolic, aspartic, phthalic, tartaric, lauric, stearic, salicylic, methanesulfonic, benzenesulfonic, sorbic, picric, benzoic, cinnamic and the like acids and salts with aminoacids like lysine, arginine, asparagine, glutamine, hystidine, serine, methionine, leucine and the like.

Representative examples of these bases are: alkali metal or alkaline-earth metal hydroxide such

sodium, potasium, and calcium hydroxide; and organic aliphatic, alicyclic or aromatic amines such as methylamine, dimethylamine, trimethylamine, and picoline.

A preferred group of compounds of the invention is represented by those compounds of formula I wherein $R^1$ and $R^2$ independently represents hydrogen, $(C_1$-$C_{12})$alkyl, $(C_4$-$C_6)$cycloalkyl, cyano $(C_1$-$C_2)$alkyl, $(CH_2)_n$OOC$(C_1$-$C_3)$alkyl wherein n represents 1 or 2, phenyl $(C_1$-$C_3)$alkyl wherein the phenyl mojety is optionally substituted with 1 or 2 substituents selected from methyl, nitro, bromo, chloro, iodo or methoxy. Another preferred group of compounds of the invention is represented by those compounds of formula I wherein $R^1$ or $R^2$ or both $R^1$ and $R^2$ represents $(C_1$-$C_4)$alkyl and A, B, and M are as defined.

A further preferred group of compounds of the invention is represented by those compounds of formula I wherein $R^1$ or $R^2$ represents $(C_1$-$C_4)$alkyl and A, B, and M represent the sugar mojeities, defined above or A, B, and M, represent hydrogen atoms.

Another group of preferred compounds is represented by those compounds of formula I wherein $R^1$ and $R^2$ or both $R^1$ and $R^2$ represents methyl or ethyl and A, B, and M represent the sugar mojeties defined above or A, B, and M represent hydrogen atoms. Preferred compounds of the invention are those compounds of formula I wherein A, B and M represents the sugar mojeties defined above or A, B and M represent hydrogen atoms. Further preferred compounds are those compounds of formula I wherein A, B and M represents the sugar mojeties defined above and the N[$(C_{10}$-$C_{11})$aliphatic acyl substituent represents 8-methylnonanoyl.

Other preferred compounds of the invention are:

- a compound of formula I wherein $R^1$ represents $(C_1$-$C_4)$alkyl, or $(C_8$-$C_{12})$alkyl and $R^2$ represents hydrogen.
- a compound of formula I wherein $R^1$ is methyl and $R^2$ is hydrogen.
- a compound of formula I wherein $R^1$ and $R^2$ are methyl.
- a compound of formula I wherein A represents hydrogen, B and M are different from hydrogen, $R^1$ represents a linear $C_{11}$ alkyl and $R^2$ represents hydrogen.
- a compound of formula I wherein A and M represent hydrogen, B is different from hydrogen, $R^1$ represents n-octyl and $R^2$ represents hydrogen.

The transformation of the free amino or non-salt compounds of the invention into the corresponding addition salts, and the reverse, i.e. the transformation of an addition salt of a compound of the invention into the non-salt or free amino form, are within the ordinary technical skill and are encompassed by the present invention.

For instance, a compound of formula I can be transformed into the corresponding acid or base addition-salt by dissolving the non-salt form in an aqueous solvent and adding a slight molar excess of the selected acid or base.

The resulting solution or suspension is then lyophilized to recover the desired salt. Instead of lyophilizing, in some instances, it is possible to recover the final salt by extraction with an organic solvent, concentration to a small volume of the separated organic phase and precipitation by adding a non-solvent.

In case the final salt is unsoluble in an organic solvent where the non-salt form is soluble it is recovered by filtration from the organic solution of the non-salt form after addition of the stoichiometric amount or a slight molar excess of the selected acid or base.

The non-salt form can be prepared from a corresponding acid or base salt dissolved in an aqueous solvent which is then neutralized to free the non-salt form.

This is then recovered for instance by extraction with an organic solvent or is transformed into another base or acid addition salt by adding the selected acid or base and working up as above.

When following the neutralization desalting is necessary, a common desalting procedure may be employed.

For example, column chromatography on controlled pore polydextrane resins (such as Sephadex L H 20) or silanized silica gel may be conveniently used.

After eluting the undesired salts with an aqueous solution, the desired product is eluted by means of linear gradient or step-gradient of a mixture of water and a polar or apolar organic solvent, such as acetonitrile/water from 50:50 to about 100% acetonitrile.

As is known in the art, the salt formation either with pharmaceutically acceptable acids (bases) or non-pharmaceutically acceptable acids (bases) may be used as a convenient purification technique.

After formation and isolation, the salt form of a compound of formula I can be transformed into the corresponding non-salt or into a pharmaceutically acceptable salt.

In some instances the acid addition salt of a compound of formula I is more soluble in water and hydrophilic solvents and has an increased chemical stability.

However, in view of the similarity of the properties of the compounds of formula I and their salts, what is said in the present application when dealing with the biological activites of the compounds of formula I

applies also to their pharmaceutically acceptable salts, and viceversa.

The compounds of the invention are useful as semi-synthetic antibacterial agents mainly active against gram positive bacteria. They also fall to be considered as growth promoters and their formulation and use for this purpose are within the scope of this invention.

The mono-and di-alkyl derivatives of the invention can be prepared according to a variety of alkylation procedures such as an alkylation with an alkyl halide in the presence of a base or a reductive alkylation with an aldehyde in the presence of a reducing agent.

A preferred procedure for preparing a mono-alkyl derivative of the invention is represented by an alkylation with an alkyl halide $R^1X$ or $R^2X$ wherein the alkyl portion is represented by an "alkyl" group which corresponds to the desired meaning of $R^1$ or $R^2$ in the final product and the halide is preferably a chloride, bromide or iodide and most preferably bromide or chloride.

The reaction occurs at about room temperature in the presence of an organic or inorganic base of medium strenght such as triethylamine, trimethylamine, tributylamine, sodium or potassium bicarbonate and the like.

Preferably, the reaction medium includes also an organic solvent capable of at least partially solubilizing the starting materials such as organic amides, alkyl ethers, ethers of glycols and polyols, phosphoramides, sulfoxides and aromatic compounds e.g., dimethylformamide, dimethoxyethane, hexamethylphosphoramide, dimethylsulfoxide, benzene, toluene and mixtures thereof.

This solvent should have a low water content (generally below about 5%).

The alkyl halide and the starting teicoplanin derivative are preferably employed in about equimolecular proportion or in a slight molar excess of the alkyl halide so that the mono-alkyl derivative is obtained in good yields.

As already said, the reaction occurs at about room temperature so that external heating is not in general necessary, even if the reaction can be carried out at temperatures of 40-70°C.

When a dialkyl derivative is desired wherein $R^1$ and $R^2$ are the same, the above reaction wherein a molar excess (3-4 times) of alkylating agent ($R^1X$ or $R^2X$) and a consequently increased amount of base is employed.

When a dialkyl derivative is desired wherein $R^1$ and $R^2$ are as defined above but different from each other, the mono-alkyl derivative is reacted with the suitable alkyl halide of formula $R^2X$ (or $R^1X$) under essentially the same conditions described above for the mono-alkylation.

Alternatively, the compound of the invention can be prepared by reductive alkylation. In this case, the teicoplanin starting material is reacted with a molar excess of a carbonylic derivative of formula $R^3R^4CO$ in the presence of a proper reducing agent.

In this formula, $R^3$ and $R^4$ independently represent hydrogen or corresponds to the $R^1$ or $R^2$ in the above formula I but with a methylene unit less than them.

As known in fact, the carbonyl group, upon reduction with proper reducing agents, gives a methylene unit. Suitable reducing agents are mixed hydrides and other reducing agents known to reduce selectively a Schiff base.

Examples of said hydrides are sodium borohydride, potassium borohydride, and sodium cyanoborohydride.

The reaction is conducted in the presence of an inert solvent such as organic amides, alkyl ethers, ethers of glycols and polyols, phosphoramides, sulfoxides and aromatic compounds e.g., dimethylformamide, dimethoxyethane, hexamethylphosphoramide, dimethylsulfoxide, benzene, toluene and mixtures thereof.

Also hydroalcoholic mixtures wherein the organic component represents preferably at least 75% of the mixture can be employed as a solvent in the present reaction. An example of such mixtures is ethanol/water, 8:2. In some instances the preferred solvent is dimethylformamide.

The reaction temperature is generally between 5°C and 40°C.

The reaction time is dependent on the other specific reaction parameters and may vary from 2 h to 48 h.

In any case, since the reaction can be followed by HPLC and other techniques the skilled man is capable of deciding when the reaction is to be considered as complete and the work up procedure may be started.

In some instances, depending on the meanings of $R^3$ and $R^4$, the above reaction may result in the preparation of the dialkyl derivatives of the invention.

In particular, when formaldehyde, acetaldehyde and 4-bromobenzaldehyde are employed, the corresponding "di-alkyl" derivative is obtained in good yields, while the "mono-alkyl" one is obtained only in very poor yields.

In addition, the sugar moiety of an alkyl compound of formula I may be selectively removed to transform it into another amide compound of formula I.

For example, an alkyl compound of formula I wherein A, B, and M represent a sugar moiety as above defined can be transformed into the corresponding compound wherein B and M are as above and A is hydrogen by means of controlled acid hydrolysis in a strong concentrated aqueous organic acid.

The concentrated organic acid in this case is preferably aqueous trifluoroacetic acid at a concentration between 75% and 95%, and the reaction temperature is preferably between 10° and 50°C.

The preferred hydrolysis conditions are represented by about 90% trifluoroacetic acid at room temperature.

The reaction time varies depending on the other specific reaction parameters but, in any case, the reaction may be monitored by TLC or preferably HPLC techniques.

An analogous selective hydrolysis is reported in European Patent Application No 84114559.2.

Similarly, alkyl compounds of formula I wherein A, B, and M represent a sugar moiety as above defined or A represents hydrogen and B and M represent sugar moieties as above defined can be transformed into the corresponding alkyl compounds of formula I wherein A and M represent hydrogen and B represent a sugar moiety as defined by means of a selective hydrolysis with a strong acid in the presence of a polar aprotic solvent selected from ethers, ketones, and mixture thereof which are liquid at room temperature.

Preferred hydrolysis conditions are in this case represented by the use of a concentrated mineral acid in the presence of an ether such as dimethoxyethane at room temperature.

Also in this case, the reaction course may be monitored by TLC or preferably HPLC. An analogous selective hydrolysis is reported in European Patent Application No. 85109495.

According to another embodiment of the present invention, an alkyl compound of formula I wherein A, B and M represents sugar moieties as defined above, an amide compound of formula I wherein A represents hydrogen and B and M represent the above defined sugar moieties, or an alkyl compound of formula I wherein A and M represent hydrogen, and B represents a sugar moiety as above defined may be transformed into the corresponding alkyl compound of formula I wherein A, B and M represents hydrogen atoms by means of a selective hydrolysis in an organic protic solvent selected from aliphatic acids and alpha-halogenated aliphatic acids which at the reaction temperature are liquids, aliphatic and cycloaliphatic alkanols which at the reaction temperature are liquids slightly mixable with water, phenylsubstituted lower alkanols wherein the phenyl moiety may optionally carry $(C_1-C_4)$alkyl, $(C_1-C_4)$alkoxy or halo rests which at the reaction temperature are liquids slightly mixable with water, and beta-polyhalogenated lower alkanols, which at the reaction temperature are liquids; in the presence of a strong acid, compatible with the solvent, selected from strong mineral acids, strong organic acids and strong acid cation exchanger resins in the hydrogen form and at a temperature between 20°C and 100°C.

In this case, the preferred hydrolysis conditions are represented by the use of a mineral acid, such as hydrochloric acid, in an haloalkanol such as trifluoroethanol, at a temperature between 65°C and 85°C.

Analogous selective hydrolysis conditions on a similar substrate are described in European Patent Application No. 84114558.4.

In the following table (Table I) the structure formulas of representative examples of compounds of the invention are reported.

TABLE I

| COMPOUND | A | B | M | $R^1$ | $R^2$ |
|---|---|---|---|---|---|
| 1 | $-GNHCOR_{(1-3)}$ | $-GNHCOCH_3$ | -M | H | $CH_3$ |
| 2 | $-GNHCOR_{(4,5)}$ | do | do | do | do |
| 3 | $-GNHCOR_{(1-3)}$ | do | do | $CH_3$ | $CH_3$ |
| 4 | $-GNHCOR_{(4,5)}$ | do | do | do | do |
| 5 | H | do | do | H | $CH_3$ |

Continued.....

TABLE I

| COMPOUND | A | B | M | $R^1$ | $R^2$ |
|---|---|---|---|---|---|
| 6 | do | do | do | $CH_3$ | $CH_3$ |
| 7 | do | do | do | H | $n-C_{12}H_{25}$ |
| 8 | H | do | H | $CH_3$ | $CH_3$ |
| 9 | do | do | do | H | $n-C_8H_{17}$ |
| 10 | do | do | do | do | $-CH_2-C_6H_5$ |

Continued....

TABLE I

| COMPOUND | A | B | M | $R^1$ | $R^2$ |
|---|---|---|---|---|---|
| 11 | do | do | do | H | $-CH_2OOC(CH_3)_3$ |
| 12 | H | H | H | $CH_3$ | $CH_3$ |
| 13 | do | do | do | $-CH_2-$ 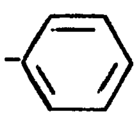 -Br | $-CH_2-$ 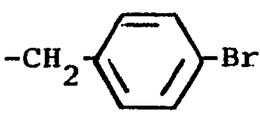 -Br |
| 14 | do | do | do | H | $n-C_3H_7$ |
| 15 | do | do | do | H | 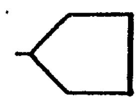 |

Continued....

TABLE I

| COMPOUND | A | B | M | $R^1$ | $R^2$ |
|---|---|---|---|---|---|
| 16 | $-GNHCOR_{(1-5)}$ | $-GNHCOCH_3$ | -M | H | $C_2H_5$ |
| 17 | do | do | do | $CH_3$ | $C_2H_5$ |
| 18 | do | do | do | $C_2H_5$ | $C_2H_5$ |
| 19 | H | do | H | H | $CH_3$ |
| 20 | H | H | H | H | $CH_2C{\equiv}N$ |

Continued....

TABLE I

| COMPOUND | A | B | M | $R^1$ | $R^2$ |
|---|---|---|---|---|---|
| 21 | H | H | H | H | $CH_3$ |
| 22 | do | do | do | H | $C_2H_5$ |
| 23 | do | do | do | $CH_3$ | $C_2H_5$ |
| 24 | do | do | do | $C_2H_5$ | $C_2H_5$ |
| 25 | $-GNHCOR_{(1-5)}$ | $-GNHCOCH_3$ | -M | H | $i-C_3H_7$ |

$R_{(1-3)}$ and $R_{(4,5)}$ designate the $C_{10}-C_{11}$ aliphatic moieties of the respective components of teicoplanin $A_2$ complex.

HPLC Analysis

The following table (Table III) reports the $R_t$ of representative examples of the compounds of the invention.
The assays were run with a HLP Hewlett - Packard 1084 apparatus equipped with UV detector (254 nm).

Columns: Stainless steel Precolumn Hibar Lichro CART packed with Lichrosorb RP-8 Merck (silanized silica gel 5 um) followed by a Hibar Lichro CART column (25 cm) pre-packed with Lichrosorb RP-8 (7 µm)
Flow rate : 1.5 ml/min
Injection volume : 30 µl

Eluents:Solution A: $CH_3CN/naH_2PO_4$ (0.02 M), 5/95 (v/v)
Solution B: $CH_3CN/NaH_2PO_4$ (0.02 M), 75/25 (v/v)

Gradient profile:

| minute: | 0 | 40 | 45 | 48 | 55 | 56 |
|---|---|---|---|---|---|---|
| % B in A | 8 | 40 | 60 | 65 | 8 | - |

TABLE III
(HPLC Analysis)

| No. COMPOUND | $t_R$ | $t_R/t_R$ L 17392 |
|---|---|---|
| 1 | 27.07-28.12*-28.85 | 1.84 |
| 2 | 31.55-32.14* | 2.08 |
| 3 | 28.13-29.03*-29.76 | 1.88 |
| 4 | 32.54-33.12* | 2.15 |
| 5 | 11.25 | 0.74 |
| 6 | 12.41 | 0.81 |
| 7 | 47.84 | 3.12 |
| 8 | 14.68 | 0.95 |
| 9 | 43.63 | 2.84 |
| 10 | 28.47 | 1.86 |
| 11 | 31.88 | 2.08 |
| 12 | 18.11 | 1.20 |
| 13 | 50.09 | 3.26 |
| 14 | 22.56 | 1.49 |
| 15 | 26.68 | 1.75 |
| Deglucoteicoplanin (standard) | 15.30 | 1.00 |

* Peak utilized for the ratio calculation.

The following table (TABLE IV) reports the acid-base tritation data of some representative compounds of the invention. The assays were carried out in Methylcellosolve $^R/H_2O$, 4/1 (v/v). A sample (10 µ mole in about 20 ml) then 0.01 N HCl (2 mol) is added and the mixture is titrated with 0.01 N KOH in the same solvent mixture.

TABLE IV

Acid base titration

| COMPOUND | Salt Form | $pK_1$ | $pK_2$ |
|---|---|---|---|
| 1 | Internal salt | 4.9 | 7.1 |
| 2 | - - - | | |
| 3 | HCL 4.8 6.9 | | |
| 4 | - - - | | |
| 5 | Internal salt | 4.9 | 7.0 |
| 6 | Internal salt | 4.8 | 6.8 |

TABLE V

IR Data ($cm^{-1}$, nujol)

| COMPOUND | νNH Glycosidic and phenolic OH | νC=O (COOH) (amide I) | δNH (amide II) | Glucoside δOH, νC-O | Phenolic νC-O |
|---|---|---|---|---|---|
| 1 | 3700-3100 | 1650 (amide I) | 1505 | 1250-1190<br>1100-930 | o.b. |
| 2 | 3700-3100 | 1725 (COOH)<br>1650 (amide I) | 1505 | 1250-1190<br>1100-930 | o.b. |
| 3 | 3700-3100 | 1725 (COOH)<br>1650 (amide I) | 1510 | 1250-1190<br>1100-930 | o.b. |
| 4 | 3700-3100 | 1725 (COOH)<br>1650 (amide I) | 1510 | 1250-1190<br>1100-930 | o.b. |

Continued....

TABLE V

IR Data ($cm^{-1}$, nujol)

| COMPOUND | ν NH Glycosidic and phenolic OH | νC=0 (COOH) (amide I) | δNH (amide II) | Glucoside δOH, νC-O | Phenolic νC-O |
|---|---|---|---|---|---|
| 5 | 3700-3100 | 1650 (amide I) | 1515 | 1250-1190<br>1100-930 | o.b. |
| 6 | 3700-3100 | 1650 (amide I) | 1515 | 1250-1190<br>1100-930 | o.b. |
| 7 | 3700-3100 | 1655 | 1515 | 1250-1190<br>1100-930 | o.b. |

Continued......

**TABLE V**

IR Data ($cm^{-1}$, nujol)

| COMPOUND | $\nu NH$<br>Glycosidic and phenolic OH | $\nu$ C=O (COOH)<br>(amide I) | $\delta NH$<br>(amide II) | Glucoside<br>$\delta_{OH}$, $\nu_{C-O}$ | Phenolic<br>$\nu_{C-O}$ |
|---|---|---|---|---|---|
| 9 | 3700-3100 | 1725 (COOH)<br>1645 (amide I) | 1515 | 1250-1190<br>1100-930 | o.b. |
| 10 | 3700-3100 | 1725 (COOH)<br>1645 (amide I) | 1515 | 1250-1190<br>1100-930 | o.b.<br>- |
| 11 | 3700-3100 | 1725 (COOH)<br>1650 (amide I) | 1510 | 1250-1190<br>1100-930 | -<br>o.b. |

Continued.....

TABLE V

IR Data ($cm^{-1}$, nujol)

| COMPOUND | $\nu$ NH<br>Glycosidic and phenolic OH | $\nu$ C=0 (COOH) (amide I) | $\delta$ NH (amide II) | Glucoside $\delta$OH, $\nu$C-O | Phenolic $\nu$C-O |
|---|---|---|---|---|---|
| 13 | 3700-3100 | 1725(COOH)<br>1645 (amide I) | 1515 | - | 1230,1060<br>1010 |
| 14 | 3700-3100 | 1650 | 1515 | - | 1005 |
| 15 | 3700-3100 | 1650 | 1515 | - | 1230<br>1060, 1010 |

Continued.....

**TABLE V**

IR Data ($cm^{-1}$, nujol)

| COMPOUND | νNH<br>Glycosidic and phenolic OH | νC=0<br>(COOH)<br>(amide I) | δNH<br>(amide II) | Glucoside<br>δOH, νC-O | Phenolic<br>νC-O |
|---|---|---|---|---|---|
| 16 | 3700-3100 | 1640 (amide I) | 1510 | 1250-1190<br>1100-930 | o.b. |
| 17 | 3700-3100 | 1655 (amide I) | 1505 | 1250-1190<br>1100-930 | o.b. |
| 18 | 3700-3100 | (COOH)<br>1655 (amide I) | 1510 | 1250-1190<br>1100-930 | o.b. |
| 19 | 3700-3100 | 1725 (COOH)<br>1645 (amide I) | 1515 | 1250-1190<br>1100-930 | o.b. |

Continued....

TABLE V

IR Data ($cm^{-1}$, nujol)

| COMPOUND | $\nu$NH Glycosidic and phenolic OH | $\nu$C=0 (COOH) (amide I) | $\delta$NH (amide II) | Glucoside $\delta$OH, $\nu$C-O | Phenolic $\nu$C-O |
|---|---|---|---|---|---|
| 20 | 3700-3100 | 1725 (COOH)<br>1650 (amide I) | 1505 | | 1060,1000 |
| 21 | 3700-3100 | 1725 (COOH)<br>1640 (amide I) | 1510 | | 1060,1005 |
| 22 | 3700-3100 | 1725 (COOH)<br>1645 (amide I) | 1510 | | 1060,1005 |

Continued...

TABLE V

IR Data ($cm^{-1}$ ,nujol)

| COMPOUND | ν NH Glycosidic and phenolic OH | ν C=0 (COOH) (amide I) | δ NH (amide II) | Glucoside δ OH, ν C-O | Phenolic ν C-O |
|---|---|---|---|---|---|
| 23 | 3700-3100 | 1645 (amide I) | 1510 | | 1060,1010 |
| 24 | 3700-3100 | 1725 (COOH)<br>1650 (amide I) | 1515 | | 1060,1010 |
| 25 | 3700-3100 | 1650 (amide I) | 1510 | 1250-1190<br>1100-930 | o.b. |

o.b.= Overlapped band

TABLE VI

UV Data ($\lambda$ max, nm)

| Solvent | Compound No. | Compound No. | Compound No. | |
|---|---|---|---|---|
| | 1 | 2 | 3 | 4 |
| Methanol | | | 280 | 280 |
| 0.1N HCl | 280 | 279 | 280 | 280 |
| Phosphate buffer pH 7.4 | 280 | 279 | 280 | 279 |
| Phosphate buffer pH 9.0 | 280 | | 280 | 280 |
| 0.1N KOH | 298 | 298 | 298 | 298 |

Continued.....

TABLE VI

UV Data ($\lambda$max, nm)

| Solvent | Compound No. | Compound No. |
|---|---|---|
| | 5 | 6 |
| Methanol | 280 | |
| 0.1N HCl | 278 | 278 |
| Phosphate buffer pH 7.4 | 278 | 278 |
| Phosphate buffer pH 9.0 | 280 | |
| 0.1N KOH | 296 | 296 |

## TABLE VI

UV Data ($\lambda$max, nm)

| Solvent | Compound No. 16 |
|---|---|
| Methanol | - |
| 0.1N HCl | 279 |
| Phosphate buffer pH 7.4 | 279 |
| Phosphate buffer pH 9.0 | - |
| 0.1N KOH | 298 |

Continued.....

Continued.....

TABLE VI

UV Data ($\lambda$max, nm)

| Solvent | Compound No. | Compound No. | |
|---|---|---|---|
| | 17 | 18 | 19 |
| Methanol | | | |
| 0.1N HCl | 279 | 280 | 279 |
| Phosphate buffer pH 7.4 | 279 | 279 | 279 |
| Phosphate buffer pH 9.0 | | | |
| 0.1N KOH | 298 | 298 | 297 |

Continued.....

TABLE VI

UV Data ($\lambda$ max, nm)

| Solvent | Compound No. | Compound No. | Compound No. | Compound No. | Compound No. |
|---|---|---|---|---|---|
| | 21 | 22 | 23 | 24 | 25 |
| Methanol | - | - | - | - | - |
| 0.1N HCl | 279 | 279 | 279 | 278 | 279 |
| Phosphate buffer pH 7.4 | 279 | 279 | 279 | 278 | 279 |
| Phosphate buffer pH 9.0 | - | - | - | - | - |
| 0.1N KOH | 297 | 297 | 297 | 297 | 298 |

Table VII reports $^1$H NMR data obtained at 250 MHz with a Bruker AM-250 Spectrometer in DMSO-$D_6$ at 20°C, at a sample concentration of 20 mg/ml (internal standard : TMS, $\delta$ = 0.00 ppm).

TABLE VII

$^{1}H$-NMR Spectra (δppm) in DMSO-$d_6$

| COMPOUND | |
|---|---|
| 1 | 0.83, 113-1.22, 1.42; 2.02 (acyl chain); 1.87 (acetylglucosamine); 2,31 (N-$CH_3$), 3.49 (mannose); 5.58 ($C_{27}$ H); 5.09 ($C_{26}$ H); 6.30-8.60, (aromatic protons) and peptidic NH's); 4.09-5.70 (peptidic CH's) |
| 2 | 0.83, 1.13-1.22, 1.43, 2.02 (acyl chain); 1.87 (acetylglucosamine); 2.49 ($N^+CH_3$); 3.48 (mannose); 5.60 ($C_{27}$ H); 5.10 ($C_{26}$ H); 4.09-5.70, (peptidic CH's); 6.20-8.60 (aromatic protons and peptidic NH's) |
| 3 | 0.83, 1.13-1.22, 1.44, 2.03 (acyl chain); 1.87 (acetylglucosamine); 2.49 ($N^+$-$CH_3$); 3.48 (mannose); 5.58 ($C_{27}$ H); 5.11 ($C_{26}$ H); 4.09-5.70, (peptidic CH's); 6.20-8.60 (aromatic CH's and peptidic NH's) |
| 4 | 0.83, 1.13-1.22, 1.44, 2.03 (acyl chain); 1.87 (acetylglucosamine); 2.49 ($N^+$-$CH_3$); 4.09-5.70 (peptidic CH's); 6.20-8.60 (aromatic CH's and peptidic NH's) |

Continued ......

TABLE VII

$^1$H-NMR Spectra (δ ppm) in DMSO-$d_6$

| COMPOUND | |
|---|---|
| 5 | 1.86 (acetylglucosamine); 2.30 (N-$CH_3$); 4.09-5.70 (peptidic CH's 5.59 (C-$_{27}$ H); 5.10 (C-$_{26}$ H); 4.09-5.70 (peptidic CH's); 6.02-8.49, (aromatic CH's and peptidic NH's) |
| 6 | 1.87 (acetylglucosamine); 2.30 ($NCH_3$); 4.09-5.70 (peptidic CH's); 5.63, (C-$_{27}$ H); 5.09 (C-$_{26}$ H); 6.00-8.60 (aromatic CH's and peptidic NH's) |
| 7 | 0.83, 1.20, 1.60 (n-lauryl chain); 1.86 (acetylglucosamine); 4.10-5.70 (peptidic CH's); 5.08 (C-$_{26}$H); 5.60 (C-$_{27}$H); 6.02-8.60 (aromatic $CH^3$s and peptidic NH's) |
| 8 | 1.88 (acetylglucosamine); 2.33 (N$\langle^{CH_3}_{CH_3}$); 4.14-5.67 (aromatic proton, peptidic CH's); 5.51 (C-$_{27}$ H); 5.06 (C-$_{26}$ H); 6.07-8.55 (aromatic CH's and peptidic NH's) |

Continued.....

TABLE VII

$^1H$-NMR Spectra (δppm) in DMSO-$d_6$

| COMPOUND | |
|---|---|
| 9 | 0.86, 1.22, 1.62 (n-octyl chain); 1.87-3.02, 3.70 (acetylglucosamine); 4.00-5.70 (peptidic CH's); 5.51 ($C-_{27}$ H'); 6.04-8.70 (aromatic protons and peptidic NH's); 8.00-10.20 (phenolic OH's) |
| 10 | 2.43 ($N^+-CH_3$)<br>$CH_3$<br>; 5.70-4.11 (peptidic CH's); 5.51 ($C-_{27}$ H); 5.09 ($C-_{26}$ H); 6.24-7.90 (aromatic protons and peptidic NH's) |
| 11 | 3.78 /$\bar{C}H_2$(-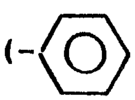-)7; 4.09-5.63 (peptidic CH's); 5.47 ($C-_{27}$ H); 5.12, ($C-_{26}$ H); 6.18-8.41 (aromatic CH's and peptidic NH's); 7.55 and 7.41(-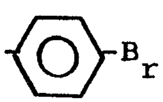-$B_r$) |

Continued......

TABLE VII

$^1$H-NMR Spectra ($\delta$ ppm) in DMSO-$d_6$

| COMPOUND | |
|---|---|
| 12 | 0.89, 1.45, 2.49 (N-propyl chain); 4.12-5.66 (peptidic CH's); 5.50 ($C_{27}$ H); 5.10 ($C_{26}$ H); 6.25-8.45 (aromatic CH's and peptidic NH's) |
| 13 | 1.50, 1.68, 5.05 (cyclopentyl); 5.67, 5.36, 4.96, 4.38, 4.34, 4.11, (peptidic CH's); 5.50 ($C_{27}$ H); 5.12 ($C_{26}$ H); 6.33-8.45 (aromatic protons and peptidic NH's) |

Continued......

TABLE VII

$^1$H-NMR Spectra (δ ppm) in DMSO-$d_6$

| COMPOUND | |
| --- | --- |
| 16 | 0.82, 140, 2.01 (acyl chain) 1.85 (acetylglucosamine); 1.05, 3.08 ($N-C_2H_5$); 4.11-5.61 (peptidic CH,s); 6.23-8.34 (aromatic protons and peptidic NH's |
| 17 | 2.25 ($N-CH_3$); 1.06, 3.06 ($N-C_2H_5$); 0.83, 1.39, 2.03 (acyl chain) 4.08-5.67 (peptidic CH's); 6.25-8.42 (aromatic protons a peptidic NH's) |
| 18 | 0.83, 1.39, 2.02 (acyl chain); 1.84 (acetylglucosamin); 1.04, 3.07 ($N-C_2H_5$); 3.47 (mannose); 4.11-5.71 (peptidic CH's); 6.29-8.38 aromatic protons a peptidic NH's) |

Continued......

TABLE VII

$^1$H-NMR Spectra (δ ppm) in DMSO-$d_6$

| COMPOUND | |
|---|---|
| 19 | 1.86 (acetylglucosamin); 2.46 (N-$CH_3$); 4.09-5.61 (peptidic CH'S); 621-8.50, (aromatic protons a peptidic NH's) |
| 20 | 4.09-5.66, (peptidic CH's); 5.08 ($C_{26}$-H); 5.48 ($C_{27}$-H); 4.84, ($CH_2$-CN) 6.22-8.44 (aromatic protons and peptidic NH's) |
| 21 | 2.48, (N-$CH_3$); 4.09-5.60 (peptidic CH's); 5.09 ($C_{26}$-H); 5.50, ($C_{27}$-H); 6.24-8.42 (aromatic protons and peptidic NH's) |
| 22. | 1.12, 2.27, (N-$C_2$-$H_5$), 4.09-5.62 (peptidic CH's); 5.09 ($C_{26}$-H); 5.61, ($C_{27}$-H); 6.23-8.44 (aromatic protons a peptidic NH's) |

Continued......

TABLE VII

$^1$H-NMR Spectra (δ ppm) in DMSO-$d_6$

| COMPOUND | |
|---|---|
| 23 | 2.28 ($N-CH_3$); 0.96, 2.52, ($N-C_2H_5$); 4.04-5.66 (peptidic CH's); 5.09 ($C_{26}$-H); 5.49 ($C_{27}$-H); 6.24-8.37 (aromatic protons a peptidic NH's) |
| 24 | 1.21, 3.14 ($N-C_2H_5$); 4.08-5.58 (peptidic CH's); 5.08 ($C_{26}$-H); 5.45 ($C_{27}$-H); 6.23-8.51 (aromatic protons and peptidic NH's) |
| 25 | 0.85, 1.35, 2.03 (acyl chain); 1.86 (acetyl glucosamine); 0.97, 1.05 ($CH_3$-isopropyl) 3.48 (mannose); 5.08 ($C_{26}$-H); 5.56 ($C_{27}$-H); 6.28-8.63 (aromatic protons); 4.09-5.66 (peptidic CH's) |

The antibacterial activity of the compounds of the invention can be demonstrated in vitro by means of standard agar-dilution tests.

Isosensitest broth (Oxoid) and Todd-Hewitt broth (Difco) are used for growing staphylococci and streptococci, respectively. Broth cultures are diluted so that the final inoculum is about $10^4$ colony forming units/ml (CFU/ml). Minimal inhibitory concentration (MIC) is considered as the lowest concentration which shows no visible growth after 18-24 h incubation at 37°C. The results of the in vitro antibacterial testing of representative compounds of formula I are summarized in Table III.

TABLE VIII

In vitro (HIC microgram/ml)

| Solvent | | Compound No. 1 | Compound No. 2 | Compound No. 3 | Compound No. 4 | Compound No. 5 | Compound No. 6 |
|---|---|---|---|---|---|---|---|
| S. Haemolyticus | 602 | n.d. | n.d. | n.d. | n.d. | n.d. | n.d. |
| S. Aureus | $10^4$ | 0.12 | 0.12 | 0.25 | 0.25 | 0.5 | 0.12 |
| TOUR<br>+ 30% bovine serum | | 0.25 | 0.5 | 0.5 | 1 | 2 | 1 |
| S. Epidermidis | ATCC 12228 | 0.25 | 0.25 | 0.25 | 0.25 | 0.5 | 0.12 |
| S. Pyogenes | C 203 | 0.06 | 0.06 | 0.06 | 0.06 | 1 | 0.5 |
| S. Pneumoniae | U C41 | 0.06 | 0.06 | 0.06 | 0.06 | 1 | 2 |
| S. Faecalis | ATCC 7080 | 0.12 | 0.12 | 0.12 | 0.12 | 4 | 4 |
| E. Coli | | > 128 | > 128 | > 128 | > 128 | > 128 | > 128 |
| P. Vulgaris | | > 128 | > 128 | > 128 | > 128 | > 128 | > 128 |
| Pseud. seruginosa | | > 128 | > 128 | > 128 | > 128 | > 128 | > 128 |

Continued....

TABLE VIII

*In vitro* (HIC microgram/ml)

| Solvent | | Compound No. 7 | Compound No. 8 | Compound No. 9 | Compound No. 10 | Compound No. 11 | Compound No. 12 |
|---|---|---|---|---|---|---|---|
| S. Haemolyticus | 602 | n.d. | n.d. | n.d. | n.d. | n.d. | n.d. |
| S. Aureus TOUR | $10^4$ | 2 | 0.25 | 0.06 | 0.25 | 2 | 0.12 |
| + 30% bovine serum | | 4 | 1 | 0.25 | 1 | 2 | 0.25 |
| S. Epidermidis | ATCC 12228 | 0.12 | 0.06 | 0.06 | 0.06 | 0.06 | 0.03 |
| S. Pyogenes | C 203 | 0.06 | 1 | 0.06 | 0.5 | 2 | 0.12 |
| S. Pneumoniae | U C41 | 0.06 | 2 | 0.06 | 1 | 2 | 0.12 |
| S. Faecalis | ATCC 7080 | 1 | 2 | 0.06 | 1 | 4 | 0.25 |
| E. Coli | | > 128 | > 128 | > 128 | > 128 | > 128 | > 128 |
| P. Vulgaris | | > 128 | > 128 | > 64 | > 128 | > 128 | > 128 |
| Pseud. seruginosa | | > 128 | > 128 | > 128 | > 128 | > 128 | > 128 |

Continued...

TABLE VIII

*In vitro* (HIC microgram/ml)

| Solvent | | Compound No. | Compound No. | Compound No. | Compound No. | Compound No. | Compound No. |
|---|---|---|---|---|---|---|---|
| | | 13 | 14 | 15 | 16 | 17 | 18 |
| S. Haemolyticus | 602 | n.d. | n.d. | n.d. | 16 | 8 | 4 |
| S. Aureus TOUR | $10^4$ | 1 | 0.06 | 0.06 | 0.5 | 0.25 | 0.25 |
| + 30% bovine serum | | 64 | 0.25 | 0.5 | 1 | 1 | 2 |
| S. Epidermidis | ATCC 12228 | 0.12 | 0.016 | 0.03 | 0.5 | 0.5 | 0.12 |
| S. Pyogenes | C 203 | 1 | 0.12 | 0.12 | 0.06 | 0.06 | 0.06 |
| S. Pneumoniae | U C41 | 0.25 | 0.12 | 0.06 | 0.12 | 0.12 | 0.12 |
| S. Faecalis | ATCC 7080 | 4 | 0.25 | 0.25 | 0.12 | 0.12 | 0.25 |
| E. Coli | | > 128 | > 64 | > 128 | > 128 | > 128 | > 128 |
| P. Vulgaris | | > 128 | > 128 | > 128 | > 128 | > 128 | > 128 |
| Pseud. seruginosa | | > 128 | > 128 | > 128 | > 128 | > 128 | > 128 |

Continued....

TABLE VIII

*In vitro* (HIC microgram/ml)

| Solvent | | Compound No. | Compound No. | Compound No. | Compound No. | Compound No. | Compound No. |
|---|---|---|---|---|---|---|---|
| | | 19 | 20 | 21 | 22 | 23 | 24 |
| S. Haemolyticus | 602 | 0.5 | 1 | 0.12 | 0.25 | 0.25 | 0.25 |
| S. Aureus | $10^4$ | 0.12 | 0.12 | 0.12 | 0.06 | 0.12 | 0.12 |
| TOUR + 30% bovine serum | | 0.5 | 4 | 0.12 | 0.25 | 0.5 | 0.5 |
| S. Epidermidis | ATCC 12228 | 0.06 | 0.12 | 0.016 | 0.03 | 0.06 | 0.06 |
| S. Pyogenes | C 203 | 0.5 | 0.12 | 0.12 | 0.06 | 0.06 | 0.12 |
| S. Pneumoniae | U C41 | 2 | 0.12 | 0.12 | 0.12 | 0.12 | 0.12 |
| S. Faecalis | ATCC 7080 | 1 | 1 | 0.12 | 0.12 | 0.12 | 0.12 |
| E. Coli | | > 128 | > 128 | > 64 | > 128 | > 128 | > 128 |
| P. Vulgaris | | > 128 | > 128 | > 128 | > 128 | > 128 | > 128 |
| Pseud. seruginosa | | > 128 | > 128 | > 128 | > 128 | > 128 | > 128 |

The $ED_{50}$ values (mg/Kg) of representative compounds of the invention in vivo tests in mice experimentally infected with S. pyogenes L 49 according to the procedure described by V. Arioli et al., Journal of Antibiotics 29, 511 (1976) are in the range of 0.17-5.0 mg/Kg s.c., and mainly between 0.17 and 1.25 mg/Kg s.c., and in the range 170-300 mg/Kg or more per os. In view of the above reported antimicrobial activity, the compounds of the present invention can effectively be employed as the active ingredient of antimicrobial preparations used in human and veterinary medicine for the prevention and treatment of infectious dieases caused by pathogenic bacteria which are susceptible to said active ingredients.

In such treatments, these compounds may be employed as such or in the form of mixtures in any proportion.

The compounds of the present invention can be administered orally, topically or parenterally wherein however, the parenteral administration is preferred. Depending on the route of administration, these compounds can be formulated into various dosage froms. Preparations for oral administration may be in the form of capsules, tablets, liquid solutions or suspensions. As known in the art the capsules the tablets may contain in addition to the active ingredient, conventional excipients such as diluents, e.g. lactose, calcium phosphate, sorbitol and the like, lubricants, e.g. magnesium stearate, talc, polyethylene glycol, binding agents, e.g. polyvinylpyrrolidone, gelatin, sorbitol, tragacanth, acacia, flavoring agents, and acceptable disintegrating and wetting agents. The liquid preparations generally in the form of aqueous or oily solutions or suspensions, may contain conventional additives such as suspending agents. For topical use the compounds of the present invention may also be prepared in suitable forms for absorption through the mucous membranes of the nose and throat or bronchial tissues and may conveniently take the form of liquid sprays or inhalants, lozenges, or throat paints.

For medication of the eyes or ears, the preparation may be presented in liquid or semi-liquid form. Topical applications may be formulated in hydrophobic or hydrophilic bases as ointment, creams, lotions, paints, or powder.

For rectal administration the compounds of the invention are administered in the form of suppositories admixed with conventional vehicles, such as, for example, cocoa butter, wax, spermaceti or polyethylenglycols and their derivatives.

Compositions for injection may take such forms as suspensions, solutions, or emulsions in oily or aqueous vehicles, and may contain formulatory agents such as suspending, stabilizing and/or dispersing agents.

Alternatively, the active ingredient may be in powder form for reconstitution at the time of delivery with a suitable vehicle, such as sterile water.

The amount of active principle to be administered depends on various factors such as the size and conditions of the subject to be treated, the route and frequency of administration, and the causative agent involved.

The compound of the invention are generally effective at a dosage comprised between about 0.5 and about 30 mg of active ingredient per Kg of body weight, preferably divided in 2 to 4 administrations per day. Particularly desirable compositions are those prepared in the form of dosage units containing from about 20 to about 300 mg per unit.

Representative examples of preparation of pharmaceutical compositions are as follows:

A parenteral solution is prepared with 100 mg of compound of Example 1 dissolved in 2 ml of sterile water for injection.

A parenteral solution is prepared with 250 mg of compound 1 to 5 hydrochloride dissolved in 3 ml of sterile water for injection.

A topical ointment is prepared with 200 mg of compound 1 or 5

3.6 g of polyethylene glycol 4000 U.S.P.

6.2 g of polyethylene glycol 400 U.S.P.

Besides their activity as medicaments, the compounds of the present invention can be used as animal growth promoters.

For this purpose, one or more of the compounds of the invention is administered orally in a suitable feed. The exact concentration employed is that which is required to provide for the active agent in a growth promotant effective amount when normal amounts of feed are consumed.

The addition of the active compounds of the invention to animal feed is preferably accomplished by preparing an appropriate feed premix containing the active compounds in an effective amount and incorporating the premix into the complete ration.

Alternatively, an intermediate concentrate or feed supplement containing the active ingredient can be blended into the feed.

The way in which such feed premixes and complete rations can be prepared and administered are described in reference books (such as "Applied ANimal Nutrition", W.H. Freedman and Co., S. Francisco, USA, 1969 or "Livestock Feeds and Feeding", O and B Books, Corvallis, Oregon, USA, 1977) and are incorporated herein bỹ references.

The following examples further illustrate the invention and must not be construed as limiting it.

EXAMPLE 1 (Procedure B)

A - Preparation of compounds 1 and 2:

a) To a stirred suspension of 10 g (about 5 mmol) of Teicoplanin $A_2$ complex (the HPLC composition was: components 1, 2 and 3 equal to 65%, components 4 and 5 equal to 35%, on the basis of the percentages of the areas of the respective peaks) in 1 l of methanol, 10 g of $NaBH_4$ powder is added portionwise at room temperature in 90 min. Then 0.4 ml (about 5 mmol) of 40% (by weight) of formaldehyde in water is added followed by 250 mg of $NaBH_4$ (powder).

The clear solution which forms is stirred at room temperature for 60 min, afterwards 1 g of $NaBH_4$ -(powder) is added.

After 120 min, additional amounts of 0.4 ml of 40% aqueous formaldehyde and 2 g of $NaBH_4$ pellets are added and the reaction mixture is stirred at room temperature overnight. Then 3 ml of 40% aqueous formaldehyde followed by 20 g of $NaBH_4$ pellets is added and stirring is continued at room temperature for 24 h. HPLC of the reaction mixture shows the presence of approximately equimolecular amounts of unreacted Teicoplanin $A_2$ complex, the mixture of compounds 1 and 2, and the mixture of compounds 3 and 4.

b) After adding 70 ml of glacial acetic acid (dropwise at room temperature) and 300 ml of water, the resulting solution is concentrated at 35°C under vacuum to evaporate most of the methanol, thus obtaining an aqueous solution (pH 4.9) which is adjusted to pH 3.0 with 10 N HCl (while cooling at 5°-10°C) and loaded on a chromatographic column containing 750 g of silanized Silica-Gel (0.06-0.2 mm, Merck Co.) in 0.001 N HCl. The column is developed with a linear gradient from 20 to 60% of $CH_3CN$ in 0.001 N HCl in 25 h at the rate of 200 ml/h, while collecting 25 ml fractions, which are checked by HPLC.

Those fractions which contain the $N^{15}$-monomethyl derivatives of teicoplanin $A_2$ components 1, 2 and 3 are combined and concentrated at 40°C under vacuum in the presence of an amount of n-butanol suitable for obtaining a dry butanolic suspension (about 100 ml) to which ethyl ether (about 200 ml) is added.

The solid which separates is collected, washed with ethyl ether and dried at 30°C in vacuo overnight, yielding 1.64 g (about 25%) of compound 1, as the hydrochloride.

Fractions containing the $N^{15}$-monomethyl derivative of Teicoplanin $A_2$ components 3 and 4 are also combined and worked up as above, yielding 0.42 g (about 12%) of compound 2, as the hydrochloride.

B) Preparation of compounds 3 and 4:

To the reaction mixture obtained by following the procedure of the above Example 1 A) point a), 40% aqueous formaldehyde (40 ml) is added followed by $NaBH_4$ pellets (45 g) while cooling to 25°C.

The reaction mixture is then diluted with 250 ml of methanol and stirred at room temperature for additional 24 h, afterwords 300 ml of water and 150 ml of glacial acetic acid are added and the resulting solution is concentrated and chromatographed according to the same procedure described in Example 1 A) a) above, thus obtaining:

5.4 g of compound 3 $N^{15}$,$N^{15}$-dimethyl derivative of Teicoplanin $A_2$ (components 1, 2, and 3, and

0.96 g of compound 4 ($N^{15}$, $N^{15}$-dimethyl derivative of Teicoplanin $A_2$ (components 4 and 5), as the hydrochlorides.

C) Preparation of compound 12

To a stirred solution of antibiotic L 17392 prepared as described in European Patent No. 0146053 (1 g, 0.8 mmol) in water (300 ml) at pH 7.8, 3.7% w/w formaldehyde (1 ml) is added.

After 15 minutes sodiumcyanoborohydride (50 mg) is added. The reaction mixture is kept at room temperature for two hours adding 1 ml formaldehyde and 50 mg of $NaBH_3CN$ every hour. (The reaction

resulted faster at pH 7/8 than at pH 4-5).

After standing overnight, the water solution is acidified to pH 2.5 with N HCl and extracted with a mixture of ethyl acetate/n-butanol 50/50 (200 ml x 2).

The organic layer is washed with water (50 ml) and the insolubiles are filtered on a paper filter.

After concentration under vacuum to a small volume (20 ml), ethyl ether (100 ml) is added and the solid which forms is collected, washed with ether and dried in vacuo at room temprature overnight to yield 950 mg of compound 12.

D) Preparation of compound 13

To a stirred solution of antibiotic L 17392 prepared as described in European Patent No. 0146053 (1 g, 0.8 mmol) in 95% EtOH (300 ml), containing sodium acetate (5 mg), p-Br-benzaldehyde (1 g) is added and the pH is adjusted to 6 by adding acetic acid.

The mixture is kept at 50°C for 3 hours adding $NaBH_3CN$ (100 mg) at one hour interval.

The mixture is then stirred overnight at room temperature. The solvent is evaporated in vacuo at 40°C and the residue is treated with acetone, filtered and washed with ethyl ether. The crude product (1.39 g) is dissolved in 100 ml of water/$CH_3CN$, 70/30 and loaded on a chromatographic column containing 100 g of silanized Silica-Gel (0.06-0.2 mm, Merck).

The column is developed with a linear gradient from 20 to 50% of acetonitrile in water, collecting 25 ml fractions which are checked by HPLC.

Those fractions containing the pure derivative of the title are are pooled and concentrated in vacuo adding n-butanol to remove water.

The residue is treated with ethyl ether, filtered, and dried in vacuo at 50°C, yielding 850 mg of compound 13.

E) Preparation of compound 14

Antibiotic L 17392 prepared as described in European Patent No. 0146053 (1 g, 0.8 mmol) is suspended in water (100 ml) and methanol (150 ml), after solubilizing at an alkaline pH (8-9) the solution is adjusted to 6 with acetic acid.

To the solution stirred at room temperture, propionaldehyde (0.3 ml) is added and after 10 minutes $NaBH_3CN$ (450 mg) worked up. After two hours, the solvent is evaporated from the reaction mass under vacuum and the resulting suspension is dissolved in a minimum amount of $CH_3CN$ and loaded on a chromatographic column containing 100 mg of silanized Silica-Gel (0.06-0.2 mm Merck).

The column is developed with a linear gradient from 0 to 20% of acetonitrile in water. Fractions containing the pure compound are pooled and concentrated after adding n-butanol (as described in the above preparation).

The residue is treated with ethyl ether, filtered and dried in vacuo overnight, yielding 500 mg of compound 14.

F) Preparation of compound 15

Antibiotic L 17392 (1 g, 0.8 mmol) is dissolved in a mixture of water (50 ml) and methanol (50 ml) at pH 9, then the pH is corected to 6 with acetic acid.

The reaction mixture is heated to 50°C and cyclopentanone (0.8 ml) and, 10 minutes later, $NaBH_3CN$ (500 mg) are added. A second portion of cyclopentanone (0.8 ml) and sodium cyanoborohydride (500 mg) is added after 1 h, and the reaction mixture is then stirred for an additional hour at 50°C overnight at room temperature.

This mixture is then acidified to pH 3.5 with HCl N, concentrated in vacuo, and the precipitate is filtered.

The crude compound (1.5 g) is dissolved in a 250 ml mixture water/$CH_3CN$, 80/20 and loaded on a column containing 200 g of silanized Silica-Gel.

The column is developed with 1.4 l of water/$CH_3CN$, 80/20 and then with water/$CH_3CN$ 75/25.

With the same recovering method reported above in Example 1E, 410 mg of compound 15 is obtained.

G) Preparation of compound 8

Antibiotic L 17046 hydrochloride (1 g, 0.7 mmol) and sodium acetate (200 mg) are dissolved in water (150 ml) and $CH_3CN$ (50 ml). 37% of formaldehyde (1 ml) is added to this solution adjusted at pH 5.4, and stirred at room temperature. After 20 minutes $NaBH_3CN$ (100 mg) is added and stirring is continued for 1 h. The excess of formaldehyde is eliminated adding a further portion of $NaBH_3CN$ (100 mg) then the reaction mixture is concentrated in vacuo to about 100 ml, methanol (50 ml) is added and the pH adjusted to 3 with NHCl.

The mixture is again concentrated to eliminate the methanol and the suspension obtained is dissolved by adding 10 ml of acetonitrile.

This solution is adsorbed on a column containing 100 g of silanized Silica-Gel that is developed with a linear gradient from 10 to 20% of aceonitrile in water. Fractions containing pure compound 8 as detected by HPLC arer pooled and evaporated after adding n-butanol.

The residue is treated with ether, the precipitate which forms filtered and dried in vacuo overnight.

Yield: 500 mg of compound 8.

H) Preparation of compounds 16-18

a) To a stirred suspension of 2 g (about 1 mmol) of Teicoplanin $A_2$ complex (whose HPLC composition is given above in A, a)) in 100 ml of methanol, 2 g of $NaBH_4$ pellets is added in one portion at room temperature. As soon as a clear solution forms (about 60 min), a solution of 0.28 ml (about 5 mmol) of acetaldehyde in 25 ml of methanol and a freshly prepared solution of 370 mg (about 10 mmol) of $NaBH_4$ in 25 ml of absolute ethanol are simultaneously added dropwise in 30 min, while maintaining the temperature between 25°C and 30°C. Stirring is continued at room temperature for 2 h HPLC analysis shows the presence of a mixture of the mono-ethyl derivative (compound 16) as the main product (about 60%), the dimethyl derivative (compound 18, about 20 %) and unreacted starting material (about 20%).

b) working up of the reaction mixture followed by column chromatography, according to the same procedure describe above in A, a), but combining all the fractions containing pure components 1 to 5, yielded the title compounds 16 (0.7 g, 35%) and 18 (0.12 g, 6%), as the hydrochlorides.

c) To the raction mixture as obtained by following the procedure of the above Example 1H, point a), additional 1.4 ml (about 25 mmol) of acetaldehyde is added under stirring at room temperature, followed by 1.1 g (about 30 mmol) of $NaBH_4$ pellets. After 3 h reation, HPLC shows the presence of a mixture of compound 18 as the main product (about 80%) and of compound 16 (aboaut 20%), the starting product being absent. Working up the reaction mixture as described above (Example 1H, b) yielded 1.1 g (55%) of compound 18 and 0.06 g (25%) of compound 16, as the hydrochlorides.

I) Preparation of compound 17

To a stirred suspension of 2 g (about 1 mmol) of compound 16 in 100 ml of methanol, 0.8 ml (about 10 mmol) of 40% (by weight) formaldehyde in $H_2O$ is added, followed by 1.1 g (about 30 mmol) of $NaBH_4$ pellets, while cooling at 25-30°C. After 2 h, 2 ml of glacial acetic acid (dropwise at room temperature) and 100 ml of $H_2O$ are added. The resulting solution is concentrated at 40°C under vacuum to evaporate most of the methanol, thus obtaining an aqueous solution (pH 3.6), which is adjusted to pH 3.0 with 1 N HCl and loaded on a chromatographic column containing 150 g of silanized Silica-gel (0.06-0.2 mm, Merck Co.) in $H_2$). The column is developed with a linear gradient form 20% to 70% of $CH_3CN$ in $H_2O$ adjusted at pH 3.2 with glacial acetic acid. Recovery is carried out as previously described in Example 1H, b) to give 1.2 g (yield about 60%) of the title compound, as the free base.

L) Preparation of compound 25

To a stirred solution of 6 h (about 3 mmol) of Teicoplanin $A_2$-complex (whose HPIC composition is given above in A, a)) in 300 ml of a mixture methanol : dimethylformamide : acetone 7:2:1 (v/v/v), 6 g of $NaBH_4$ pellets is added at 25°-30°C. After 2 h at room temperature, 120 ml of acetone is added and stirring is continued for 3 h, afterwards the reaction mixture is cooled at 10°C and additional 6 g of $NaBH_4$ pellets is added, while maintaining the temperature between 15°C and 25°C. After 60 min, the reaction is completed,

and the reaction mass is treated with 30 ml of glacial acetic acid and concentrated to a small volume (about 30 ml) at 40°C under reduced pressure. On adding ethyl ether (about 170 ml) a solid separates which is collected by filtration and re-dissolved in 500 ml of water. The resulting solution is loaded on a column of 800 g of silanized Silica-gel (0.06-0.2 mm; Merck Co.) in water. The column is then developed with 1 liter of water, then with a linear gradient from 100% of a solution of 15 ml of glacial acetic acid in water to 80% of acetonitrile in water, in 10 h at a rate of 400 ml/h, while collecting 25 ml fractions. Those fractions containing (HPLC) the pure components of compound 25 are pooled and enough n-butanol is added to obtain, after concentration at 45°C under vacuum, a cloudy dry butanolic solution (about 50 ml). On adding ethyl acetate (about 450 ml) a solid separates which is collected by filtration, washed with ethyl ether (about 200 ml) and dried at room temperature in the air for 3 days, to give 4.8 g (about 78% yield) of pure title compound, as the free base.

EXAMPLE 2 (Procedure C)

A) Preparation of compound 5

A solution of 1 mmol of $N^{15}$-methyl Teicoplanin $A_2$ complex (a component thereof or a mixture of said components in any proportion) obtained essentially by following the procedure of the foregoing Example 1 A in 50 ml of 90% aqueous trifluoroacetic acid (TEA) is stirred at room temperature for 90 min, then the solvent is completely evaporated at 30°C under vacuum.

Obtaining oily residue is triturated with ethyl ether, the solid which form is collected, washed with ethyl ether and dried at 30°C in vacuo overnight, yielding crude compound 5, as the trifluoroacetate.

This crude powder is dissolved in 100 ml of a mixture $CH_3CN:H_2O$, 1:1 (v/v) and the resulting solution is adjusted to pH 6.3 with 1N NaOH.

Most of the acetonitrile is then evaporated at room temperature under vacuum to obtain an aqueous suspension which is adjusted to pH 5.6 with 0.1N NCl.

The solution which precipitates is collected by filtration, washed with $H_2O$ and dried at 40°C in vacuo (over $P_2O_5$) overnight, yielding 1.06 g (about 67%) of compound 5 as the internal salt. This internal salt may be transformed into the corresponding hydrochloride by dissolving it (0.5 mmol) in a mixture of (60 ml) $CH_3CN$ : 005 N HCl : n-$C_4H_9OH$, 1:1:2 (v/v/v), under stirring at room temperature.

After concentration to a small volume (about 5 ml) at 40°C under vacuum, ethyl ether (about 25 ml) is added and the solid which separates is collected, washed with ethyl ether and dried at 40°C in vacuo overnight, yielding compounds 5 (about 90%) as the hydrochloride.

B) Preparation of compound 6

By following the procedure of the above Example 2 A but starting from 1 mmole of $N^{15}$, $N^{15}$-dimethyl Teicoplanin $A_2$ complex (a component thereof or a mixture of said components in any proportion obtained essentially following the procedure of Example 1 A), 1.18 g (about 74%) of compound 6 as the internal salt which may be then transformed into the corresponding hydrochloride (yield = 95%) is obtained.

EXAMPLE 3 (Procedure A)

A) Preparation of compound 7

A solution of 2.5 mmol of antibiotic L 17054, 0.5 ml (about 3.5 mmol) of triethylamine (TEA) and 2.6 mmol of lauryl bromide in 40 ml of DMF is stirred at room temperature for 96 h. By adding ethyl ether (about 300 ml) a solid separates, which is collected by filtration and re-dissolved in 200 ml of $CH_3OH$.

Water (1 l) is then added and the resulting solution is adjusted to pH 9.2 with 1N NaOH and extracted with ethyl acetate (1 l).

The organic layer is discarded, while the aqueous one is brought to pH 2.5 with 1N HCl and re-extracted twice with a mixture (2 x 1 l) ethyl acetate : n-butanol, 7:3 (v/v).

The organic layer is separated, washed with 600 ml of $H_2O$ (2 x 300 ml), and concentrated to a small

volume (about 50 ml).

By adding ethyl ether (about 250 ml) a solid separates, which is collected by filtration, washed with ethyl ether and dried at 40°C in vacuo overnight, yielding the title compound as the hydrochloride ($N^{15}$-lauryl antibiotic L 17054, hydrochloride), 1.04 g (yield about 25%).

B) Preparation of compound 9:

By essentially following the procedure of the above Example 3 A but starting from 2.5 mol of antibiotic L 17046 and 2.6 mmol of n-octyl bromide the $N^{15}$-octyl derivative of antibiotic L 17046 is obtained (2.48 g; yield about 65%)

C) Preparation of compound 10

By essentially following the procedure of the above Example 3 A but starting from 2.5 mol of antibiotic L 17046 and 2.6 mmol of benzyl bromide the $N^{15}$-benzyl derivative of antibiotic L 17046 is obtained (2.07 g; about 55%).

D) Preparation of compound 11

To a stirred solution of 3.5 g (2.5 mmol) of antibiotic L 17046 and 0.5 ml (about 3.5 mmol) of TEA in 30 ml of dry DMF, a solution of 0.74 ml (about 5 mmol) of pivaloyloxymethyl chloride in 20 ml of dry DMF is added dropwise at room temperature.

After stirring at room temperature overnight, additional 0.15 ml of TEA followed by 0.4 ml of pivaloyloxymethyl chloride is added and the mixture is stirred for 18 h.

After adding 500 ml of $H_2O$, the resulting solution is extracted with 1 l of a mixture ethyl acetate : n-butanol, 9:1 (v/v)

The organic extract is discarded and the aqueous layer is re-extracted with 800 ml of n-butanol.

This organic layer is separated, washed sequentially with 300 ml of 0.01N HCl and 300 ml of $H_2O$, and then concentrated to a small volume (about 150 ml) at 40°C under vacuum.

The cloudy solution which forms is filtered and 300 ml of a mixture ethyl acetate : ethyl ether, 3:1 (v/v) is added to the clear filtrate.

After standing 48 h at room temperature, the solid which separates is collected by filtration and re-dissolved in 20 ml of $CH_3OH$.

The methanolic solution is then diluted with 150 ml of $H_2O$ and extracted with 200 ml of a mixture ethyl acetate : n-butanol, 85:15 (v/v).

The organic layer is separated and concentrated to a small volume (about 50 ml) at 35°C under vacuum. By adding 100 ml of a mixture ethyl acetate : ethyl ether, 9:1 (v/v), a solid separates which is collected, washed with ether and dried overnight in vacuo at room temperature, yielding 0.96 g (about 25%) of the title compound, as the free base.

E) Preparation of compound 20

To a stirred solution of 1.2 g (1 mmol) of antibiotic L 17392 (deglucoteicoplanin) in 30 ml of DMF, 3.5 ml of chloroacetonitrile and 0.35 ml (about 2.5 mmol) of TEA are added at room temperature (about 20°C). After 18 h, solvents are evaporated at 40°C under vacuum and the residue is slurried with 3 ml of $H_20$, then is collected and dried at 35°C in vacuo overnight, yielding 0.96 g of the cyanomethyl ester derivative of the title compound, which is re-dissolved in 30 ml of $THF/H_2O$, 2/1. 0.5 ml of TEA is added to the resulting solution. After stirring at room temperature overnight, 30 ml of n-butanol and 5 ml of 1 H NCl are added. Solvents are evaporated at 30°C under vacuum and the residue is dissolved in 100 ml of $H_2O/CH_3CN$, 8/2. The resulting solution is then loaded on a column containing 100 g of silanized Silica-gel (0.06-0.2 mm, Merck).

Following the recovery method reported above in Example 1D, 0.35 g (about 28% yield) of compound 20 is obtained.

EXAMPLE 4 (Preparation of the sodium or potassium salts of the compounds of the invention)

The compounds of the invention (e.g. the compounds of the foregoing examples) can also be obtained as sodium or potassium salts.

In this case 1 mmol of the corresponding hydrochloride is dissolved in 100 ml of a mixture $CH_3CN:H_2O$, 1:1 (v/v). The organic solvent is evaporated ar room temperature under vacuum and the cloudy solution which forms is brought to pH 6.5 with 1N NaOH.

The solid which separates is collected by filtration and re-dissolved in a mixture (20 ml) $DMF:H_2O$, 1:1 (v/v).

After adding 25 ml of n-butanol and 1 ml of 1 N NaOH (or KOH), the solution is concentrated to a small volume (about 10 ml) at 30°C under vacuum.

By adding ethyl ether a solid separates which is collected, washed with abs. ethanol, then with ethyl ether and dried at 40°C in vacuo overnight, yielding the corresponding sodium or potassium) salt, (yields > 80%).

EXAMPLE 5 (Procedure D)

Preparation of compound 19

A stirred suspension of 3 g (about 1.5 mmol) of compound 1 (or compound 2, or a mixture thereof) in 100 ml of a saturated solution of HCl in 1,2-dimethoxyethane is heated at 50°C for 3 h, while bubbling dry HCl. The solvent is evaporated at 50°C under reduced pressure and the residue is dissolved in 200 ml of $H_2O/CH_3CN$, 95/5. The resulting solution is adjusted to pH 3.6 with 1 N NaOH and loaded on a column of 150 g of silanized Silica-gel (0.06-0.2 mm, Merck Co.) in $H_2O$. The column is developed with a linear gradient from 5% to 30% of $CH_3CN$ in $H_2O$, in 15 h at the rate of 200 ml/h, while collecting 15 ml fractions, which are checked by HPLC. Those fractions containing the pure title compound are combined and concentrated at 40°C under vacuum in the presence of n-butanol obtaining a dry butanolic suspension (about 30 ml) to which ethyl ether (about 70 ml) is added. The solid which separates is collected, washed with ethyl ether and dried at 40°C in vacuo overnight, yielding 0.88 g (37%) of compound 19, as the hydrochloride.

EXAMPLE 6 (Procedure E)

Preparation of compounds 21, 22, 23 and 24

a) A stirred suspension of 2 g (about 1 mmol) of compound 1 (or compound 2, or a mixture thereof) in 3 ml of 0.5 M HCl solution in absolute 2,2,2-trifluoroethanol is heated at 80°C for 12 h, while continuously bubbling anhydrous HCl. After cooling to room temperature, the reaction mixture is filtered and the collected solid is dissolved in 200 ml of $H_2O/CH_3CN$, 85/15. The resulting solution is loaded on a column of 200 g of silanized Silica-gel (0.06-0.2 mm, Merck Co.) which is then developed with a linear gradient from 10% to 50% of $CH_3CN$ in 0.001 NHCl in 10 H at the rate of 200 ml/h, while collecting 10 ml fractions. Those containing pure (HPLC) title compound are pooled and solvents are evaporated at 40°C under vacuum to give 1 g (about 80% yield) of compound 21, as the hydrochloride.

b) By following the same procedure as described above, compounds 22 (77% yield), 23 (71%) and 24-(84%) are respectively obtained, as the hydrochlorides, from compounds 16, 17 and 18.

## Claims

1. $N^{15}$-alkyl and $N^{15}$,$N^{15}$-dialkyl derivatives of teicoplanin compounds having the following formula:

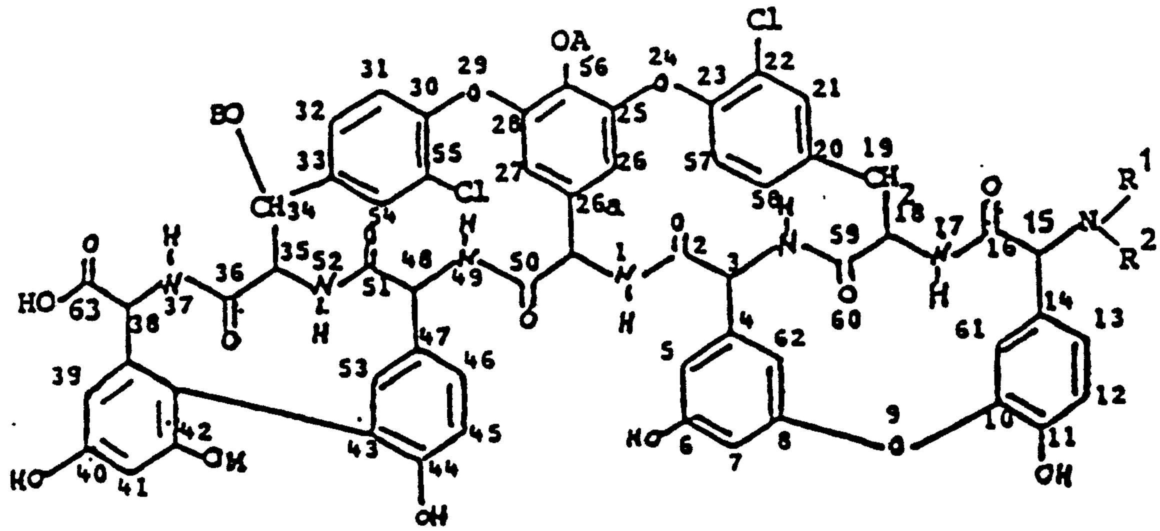

wherein

$R^1$ and $R^2$ independently represents hydrogen; $(C_1$-$C_{12})$alkyl; $(C_4$-$C_7)$cycloalkyl; cyano $(C_1$-$C_3)$alkyl; --$(CH_2)_n$-OOC-$(C_1$-$C_6)$alkyl; wherein n is an integer selected from 1, 2, 3 and 4; phenyl $(C_1$-$C_4)$alkyl wherein the phenyl group is optionally substituted in the position ortho, meta and/or para with 1 to 3 groups selected from $(C_1$-$C_4)$alkyl, nitro, bromo, chloro, iodo, $(C_1$-$C_4)$alkoxy, and phenyl; with the proviso that $R^1$ and $R^2$ may not simultaneously represent hydrogen and when one between $R^1$ and $R^2$ represents $(CH_2)_n$-OOC-$(C_1$-$C_6)$-alkyl the other must represent hydrogen;

A represents hydrogen or -N[$(C_{10}$-$C_{11})$aliphatic acyl]-β-D-2-deoxy-2-amino-glycopyranosyl,

B represents hydrogen or N-acetyl-β-D-2-deoxy-2-amino-glucopyranosyl,

M represents hydrogen or α-D-mannopyranosyl, with the proviso that B represents hydrogen only when A and M are simultaneously hydrogen and M represents hydrogen only when A is hydrogen; and the addition salts thereof.

2. A compound of claim 1 wherein $R^1$ represents $(C_1$-$C_4)$alkyl, or $(C_8$-$C_{12})$alkyl and $R^2$ represents hydrogen.

3. A compound of claim 1 wherein $R^1$ is methyl and $R^2$ is hydrogen.

4. A compound of claim 1 wherein $R^1$ and $R^2$ are methyl.

5. A compound of claim 1 wherein A represents hydrogen, B and M are different from hydrogen, $R^1$ represents a linear $C_{11}$ alkyl and $R^2$ represents hydrogen.

6. A compound of claim 1 wherein A and M represent hydrogen, B is different from hydrogen, $R^1$ represents n-octyl and $R^2$ represents hydrogen.

7. A process for preparing a compound of claims 1, 2, 3, 4, 5 or 6 which comprises reacting a teicoplanin starting material selected from a compound of the above Formula I wherein $R^1$ and $R^2$ are hydrogen, hydroxy, A represents hydrogen or -N[$(_{10}$-$C_{11})$aliphatic acyl]-β-D-2-deoxy-2-amino-glucopyranosyl, B represent hydrogen or N-acetyl-β-D-2-deoxy-2-amino glucopyranosyl, M represents hydrogen or α-D-mannopyranosyl, with the proviso that B may represent hydrogen only when A and M are simultaneously hydrogen and M may represent hydrogen only when A is hydrogen, a salt thereof, or a mixture thereof in any proportion with:

a) an alkyl halide of formula $R^1X$ or $R^2X$, wherein $R^1$ and $R^2$ are as defined and X represents chloro or bromo in the presnece of a base and possibly of an organic solvent

b) alternatively, a molar excess of a carbonyl compound of formula $R^3$ $R^4CO$ wherein $R^3$ and $R^4$ independently represents hydrogen or an "alkyl-type" substituent having a terminal methylene unit less than a corresponding "alkyl" substituent of $R^1$ and $R^2$, respectively, in the presence of a reducing agent such as a mixed hydride.

8. A pharmaceutical composition comprising a compound of claims 1, 2, 3, 4, 5 or 6 in admixture with a pharmaceutically acceptable acid.

9. Use of a compound of claim 1, 2, 3, 4, 5 or 6 for preparing a medicament for antimicrobial use.

10. A compound of claim 1, 2, 3, 4, 5 or 6 for use as a medicine.

CLAIMS FOR THE FOLLOWING CONTRACTING STATES: GR, ES.

1. A process for preparing $N^{15}$-alkyl and $N^{15}$, $N^{15}$-dialkyl derivatives of teicoplanin compounds having the following formula:

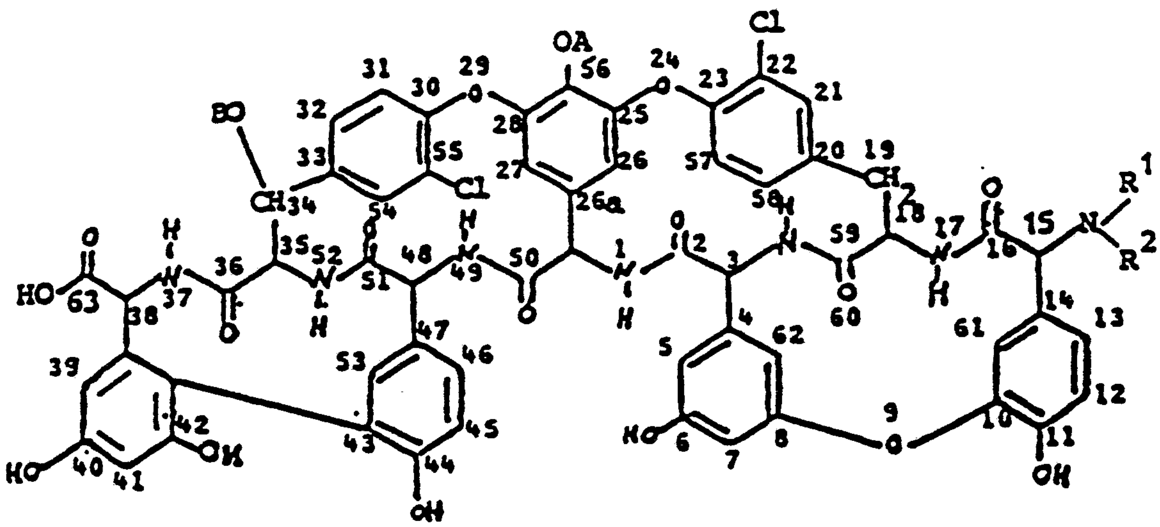

wherein

$R^1$ and $R^2$ independently represents hydrogen; $(C_1-C_{12})$alkyl; $(C_4-C_7)$cycloalkyl; cyano $(C_1-C_3)$alkyl; --$(CH_2)_n$-OOC-$(C_1-C_6)$alkyl; wherein n is an integer selected from 1, 2, 3 and 4; phenyl $(C_1-C_4)$alkyl wherein the phenyl group is optionally substituted in the position ortho, meta and/or para with 1 to 3 groups selected from $(C_1-C_4)$alkyl, nitro, bromo, chloro, iodo, $(C_1-C_4)$alkoxy, and phenyl; with the proviso that $R^1$ and $R^2$ may not simultaneously represent hydrogen and when one between $R^1$ and $R^2$ represents $(CH_2)_n$-OOC-$(C_1-C_6)$-alkyl the other must represent hydrogen;

A represents hydrogen or -N[$(C_{10}-C_{11})$aliphatic acyl]-beta-D-2-deoxy-2-amino-glycopyranosyl,

B represents hydrogen or N-acetyl-beta-D-2-deoxy-2-amino-glucopyranosyl,

M represents hydrogen or alfa-D-mannopyranosyl, with the proviso that B represents hydrogen only when A and M are simultaneously hydrogen and M represents hydrogen only when A is hydrogen; and the addition salts thereof, which comprises reacting a teicoplanin starting material selected from a compound of the above Formula I wherein $R^1$ and $R^2$ are hydrogen, hydroxy, A represents hydrogen or -N[$(_{10}-C_{11})$aliphatic acyl]-beta-D-2-deoxy-2-amino-glucopyranosyl, B represent hydrogen or N-acetyl-beta-D-2-deoxy-2-amino-glucopyranosyl, M represents hydrogen or alfa-D-mannopyranosyl, with the proviso that B may represent hydrogen only when A and M are simultaneously hydrogen and M may represent hydrogen only when A is hydrogen, a salt thereof, or a mixture thereof in any proportion, with:

a) an alkyl halide of formula $R^1X$ or $R^2X$, wherein $R^1$ and $R^2$ are as defined and X represents chloro or bromo in the presence of a base and possibly of an organic solvent

b) alternatively, a molar excess of a carbonyl compound of formula $R^3$ $R^4CO$ wherein $R^3$ and $R^4$ independently represents hydrogen or an "alkyl-type" substituent having a terminal methylene unit less than a corresponding "alkyl" substituent of $R^1$ or $R^2$, respectively, in the presence of a reducing agent such as a mixed hydride.

2. A process according to claim 1 for preparing a compound wherein $R^1$ represents $(C_1-C_4)$alkyl, or $(C_8-C_{12})$alkyl and $R^2$ represents hydrogen.

3. A process according to claim 1 for preparing a compound wherein $R^1$ is methyl and $R^2$ is hydrogen.

4. A process according to claim 1 for preparing a compound wherein $R^1$ and $R^2$ are methyl.

5. A process according to claim 1 for preparing a compound wherein A represents hydrogen, B and M are different from hydrogen, $R^1$ represents a linear $C_{11}$ alkyl and $R^2$ represents hydrogen.

6. A process according to claim 1 for preparing a compound wherein A and M represent hydrogen, B is different from hydrogen, $R^1$ represents n-octyl and $R^2$ represents hydrogen.

7. Use of a compound of claim 1, 2, 3, 4, 5 or 6 for preparing a medicament for antimicrobial use.

European Patent Office

# EUROPEAN SEARCH REPORT

Application Number

EP 88 10 0763

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 146 053 (LEPETIT)<br>* Page 1, line 5 - page 4, line 20; page 29, lines 29-31 *<br>--- | 1,9 | C 07 K 9/00<br>C 07 K 7/06<br>A 61 K 37/02 |
| Y | EP-A-0 132 116 (SMITHKLINE)<br>* Page 2, lines 5-34; page 15, lines 25-29; page 16, lines 21-26 *<br>--- | 1,9 | |
| Y | EP-A-0 201 251 (ELI LILLY)<br>* Page 2, column 2, line 16 - page 3, column 4, line 27; page 18, column 33,34, lines 1-27; page 19, column 35, lines 42-47 *<br>--- | 1,9 | |
| P,Y | THE JOURNAL OF ANTIBIOTICS, vol. 40, no. 7, July 1987, pages 970-990; S.B. CHRISTENSEN et al.: "Parvodicin, a novel glycopeptide from a new species, actinomadura parvosata: discovery, taxonomy, activity and structure elucidation"<br>* Page 984, figure 4; page 986, table 6 *<br>----- | 1,9 | TECHNICAL FIELDS SEARCHED (Int. Cl.4)<br>C 07 K 9/00<br>C 07 K 7/00<br>A 61 K 37/00 |

The present search report has been drawn up for all claims

| Place of search | Date of completion of the search | Examiner |
|---|---|---|
| THE HAGUE | 23-03-1988 | BRENNAN J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P0401)